(19) Europäisches Patentamt
European Patent Office
Office européen des brevets

(11) **EP 2 890 318 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(21) Anmeldenummer: **13752577.0**

(22) Anmeldetag: **20.06.2013**

(51) Int Cl.:
*A61B 18/12* (2006.01)     *A61B 18/04* (2006.01)
*H05H 1/30* (2006.01)     *A61L 2/00* (2006.01)

(86) Internationale Anmeldenummer:
**PCT/EP2013/001817**

(87) Internationale Veröffentlichungsnummer:
**WO 2014/032747 (06.03.2014 Gazette 2014/10)**

(54) **VORRICHTUNG ZUR BEHANDLUNG VON BIOLOGISCHEM GEWEBE MIT EINEM NIEDERDRUCKPLASMA**

APPARATUS FOR TREATING BIOLOGICAL TISSUE USING A LOW-PRESSURE PLASMA

DISPOSITIF DE TRAITEMENT D'UN TISSU BIOLOGIQUE AU MOYEN D'UN PLASMA BASSE PRESSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.08.2012 DE 102012017210**
**21.12.2012 DE 102012025082**

(43) Veröffentlichungstag der Anmeldung:
**08.07.2015 Patentblatt 2015/28**

(73) Patentinhaber: **NorthCo Ventures GmbH & Co. KG**
**90491 Nürnberg (DE)**

(72) Erfinder:
• **SRB, Josef**
**38801 Blatna (CZ)**

• **KOROUS, Josef**
**38801 Blatna (CZ)**
• **HINTERKOPF, Jan**
**52070 Aachen (DE)**

(74) Vertreter: **Bittner, Bernhard**
**Hannke Bittner & Partner**
**Patent- und Rechtsanwälte mbB**
**Neustadt 8**
**56068 Koblenz (DE)**

(56) Entgegenhaltungen:
**EP-A1- 0 837 622      EP-A2- 1 148 770**
**DE-B3-102005 000 950     FR-A- 1 119 719**
**US-A1- 2012 176 724**

**Beschreibung**

Die Erfindung betrifft eine Vorrichtung zur Behandlung von biologischem Gewebe mit einem Niederdruckplasma nach dem Patentanspruch 1.

Dass Plasmen antimikrobielle Eigenschaften besitzen, ist bekannt. Die Ursachen der antibakteriellen Wirkung eines Plasmas liegen in Hitze, Austrocknung, Scherspannung, UV-Strahlung, freie Radikale und Ladungen. Bei Niederdruckplasmen, die auch kalte Plasmen genannt werden, spielt die Hitze eine untergeordnete Rolle, da diese Plasmen bei Raumtemperatur betrieben werden. In solchen Niederdruckplasmen entstehen besonders reaktive Partikel, wie beispielsweise verschiedene Sauerstoff- oder Stickstoffspezies, die eine ausreichend hohe Lebensdauer aufweisen, um bei einer indirekten Exposition organische Verbindungen zu schädigen. Zu diesen Partikeln zählen unter anderem atomarer Sauerstoff, Superoxidradikale, Ozon, Hydroxylradikale, Stickstoffmonooxid und Stickstoffdioxid. Diese Partikel zeigen eine zerstörerische Wirkung auf unterschiedlichste Zellkomponenten.

Werden Zellwände von Bakterien, Keimen, Viren, Pilzen oder anderen vergleichbaren Mikroorganismen dem Plasma direkt ausgesetzt, so laden sich diese aufgrund des Beschusses mit den im Plasma vorhandenen Elektronen negativ auf. Aufgrund der elektrostatischen Abstoßung führt dies zu mechanischen Spannungen bis hin zur Überschreitung der Zugfestigkeit und der Zerstörung der Zellwand. Aber nicht nur mechanische Verspannungen aufgrund der Ladung können die Zellwände zerstören, sondern auch die Störung des Ladungsgleichgewichts der Zellwand durch verschiedene, weitere elektrostatische Wechselwirkungen und der Elektrolyse, z. B. durch Änderung der Permeabilität der Zellwände. Ein Mechanismus zur Inaktivierung von Mikroorganismen ergibt sich auch aus den sehr energiereichen Ionen, die in kapazitiv gekoppelten Systemen weit über 100 eV aufweisen können. Ein Beschuss mit solchen Spezies kann die strukturelle Integrität der Zellen verändern bzw. sie zerstören; allerdings ist eine Vorrichtung zur Erzeugung solcher Ionenstrahlen aufwendig und nur mit sehr hohem apparativen Aufwand zur Behandlung von lebendem biologischen Gewebe, insbesondere menschlichen oder tierischen Geweben, geeignet.

Niederdruckplasmen sind daher besonders gut zur Behandlung von Gewebe von Menschen oder Tieren, insbesondere von Hautoberflächen, offenen Wunden, des Zahnfleischs, der Mundhöhle oder dergleichen geeignet, um eine Desinfektion des Gewebes, insbesondere die Abtötung von Bakterien, Keimen, Viren, Pilzen oder anderer vergleichbarer Mikroorganismen, die in oder auf dem Gewebe platziert sind, zu erreichen.

Die Offenlegungsschrift EP 0837622 A1 offenbart den Gegenstand des Oberbegriffs des Patentanspruchs 1.

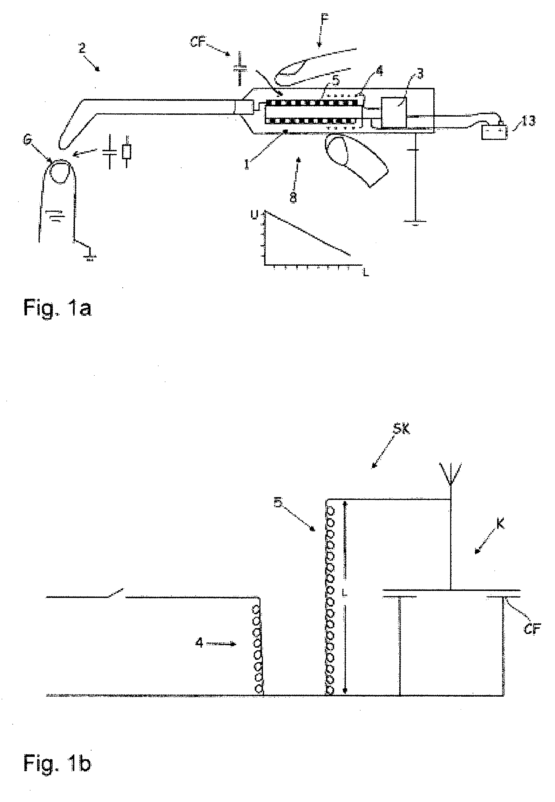

Aus der DE 10 2005 000 950 B3 ist eine Vorrichtung und ein Verfahren zur Behandlung von biologischem Gewebe mit Ozon bekannt. Diese Vorrichtung besteht im Wesentlichen aus einem mittels einer Steuereinrichtung in Spannung und/oder Stromstärke regelbaren Transformator zur Erzeugung spezieller gerichteter Spannungs- oder Stromimpulse verschiedenster Charakteristik mit oder ohne Gleichspannungsanteil. Der Gleichspannungsanteil wird dabei durch zusätzliche Elektroden am zu behandelnden biologischen Gewebe mithilfe einer externen Spannungsquelle oder Schaltung aufgebaut. Die Primärspule des Transformators ist die von hochfrequentem Wechselstrom durchflossene Spule eines gedämpften Schwingungskreises. Die Sekundärspule bildet zusammen mit dem aufzuladenden Kondensator einen Schwingkreis, dessen Frequenz mit der des Transformators übereinstimmt. Als Stromquelle dient oftmals ein Resonanztransformator. Die Schwingungsfrequenz an der Entladungsstrecke liegt z. B. in der Größenordnung 100 kHz. Bei derartigen Frequenzen sind die über die Entladungsstrecke fließenden Ströme gering und für organisches Gewebe unschädlich. Um eine gute magnetische Kopplung zwischen Primärspule und Sekundärspule zu erreichen, ist der Abstand zwischen ihnen gering. Die Spannung steigt dabei über die Spulenlänge in Richtung der Sonde an, so dass am Ende der Spulen die Gefahr eines Funkenüberschlages zwischen den Spulen nicht ausgeschlossen werden kann. Diese Gefahr wird auch noch dadurch erhöht, dass der Benutzer eine zusätzliche Kapazität bildet, die den Schwingkreis aus Sekundärspule und einer dazugehörigen Kapazität stört, so dass ein Funkenüberschlag zwischen den Spulen wahrscheinlicher wird. Diese Gefahr erhöht sich nochmals, wenn Primär- und Sekundärspule wie beispielsweise bei den Gegenständen der DE 36 18 412 A1 und der WO 2006/119997 A1 unterschiedliche Längen aufweisen, wie dies auch anhand der Figuren 1a und b nochmals verdeutlicht ist. Figur 1 b zeigt dabei ein Ersatzschaltbild der Figur 1a und veranschaulicht nochmals die Änderung der Gesamtkapazität K des Schwingkreises SK durch die Kapazität CF des Fingers F des Benutzers, wobei in Figur 1a in einem Diagramm die Spannung U über die Länge L der Sekundärspule 5 schematisch aufgetragen ist.

Es ist daher Aufgabe der Erfindung, eine Vorrichtung zur Behandlung von biologischem Gewebe mit einem Niederdruckplasma mit den Merkmalen des Oberbegriffs des Patentanspruchs 1 derart weiterzubilden, dass solche Funkenüberschläge zwischen Primär- und Sekundärspule nahezu ausgeschlossen sind.

Vorrichtungsmäßig wird diese Aufgabe durch eine Vorrichtung mit allen Merkmalen des Patentanspruchs 1 gelöst. Vorteilhafte Ausgestaltungen der Erfindung finden sich in den unabhängigen Patentansprüchen 1 und 12 abhängigen Patentansprüchen. Die erfindungsgemäße Vorrichtung zur Behandlung von biologischem Gewebe mit einem Niederdruckplasma enthält im Wesentlichen

einen Transformator zur Erzeugung eines hochfrequenten elektromagnetischen Feldes,

eine Sonde, welche mit dem Transformator elektrisch koppelbar ist und

einer Steuerungseinrichtung zur Steuerung des durch den Transformator erzeugten hochfrequenten elektromagnetischen Feldes, wobei

der Transformator eine Primärspule und eine koaxial dazu angeordnete Sekundärspule aufweist und wobei sich der Zwischenraum zwischen Primärspule und Sekundärspule im Überlappungsbereich der beiden Spulen von einem ersten Abstand zu einem zweiten größeren Abstand in Richtung einer Kupplung für die Sonde vergrößert, wobei die Primärspule konisch koaxial über der Sekundärspule angeordnet ist. Durch diese spezielle Ausgestaltung der erfindungsgemäßen Vorrichtung, insbesondere des Transformators der erfindungsgemäßen Vorrichtung ist es erreicht, dass trotz steigender Spannung über die Länge der Spulen aufgrund des sich nun vergrößernden Abstandes zwischen den Spulen das Funkenüberschlagsrisiko minimiert ist. Die zwischen den Spulen anliegende Spannung ist in keinem Bereich mehr hoch genug, um einen Funkenüberschlag zwischen Primär- und Sekundärspule zu generieren.

Vorteilhafterweise umfasst der Transformator ein Transformatorgehäuse, welches eine der Kupplung für die Sonde gegenüberliegende Kupplung zum elektrischen/elektronischem Anschluss der Steuereinrichtung aufweist, wobei das Transformatorgehäuse vorzugsweise als Handgriff ausgebildet und entsprechend ergonomisch geformt ist. Diese Maßnahme zielt auf eine kompakte Bauweise der gesamten erfindungsgemäßen Vorrichtung, da sowohl der Transformator selbst als auch die Steuerungseinheit innerhalb des Transformatorgehäuses anordnenbar ist. Lediglich die Sonde zur Behandlung des biologischen Gewebes und gegebenenfalls eine externe Energiequelle zur Energieversorgung der erfindungsgemäßen Vorrichtung sind nicht innerhalb des Transformatorgehäuses angeordnet. Die ergonomische Ausgestaltung des Transformatorgehäuses als Handgriff, der in seiner Grundform zylindrisch ausgebildet ist, erlaubt ferner eine angenehme und sichere Handhabung der erfindungsgemäßen Vorrichtung durch den Anwender.

Aus den gerade genannten Gründen der kompakten Bauweise und der einfachen, sicheren und angenehmen Handhabung der erfindungsgemäßen Vorrichtung ist daher nach einem vorteilhaften Gedanken der Erfindung die Steuereinrichtung in dem Transformatorgehäuse angeordnet.

Allerdings kann es für bestimmte Anwendungen sinnvoll sein, die Steuereinrichtung außerhalb des Transformatorgehäuses anzuordnen. Insbesondere dann, wenn sehr filigrane Behandlungen durchgeführt werden müssen, bei denen zusätzliches Gewicht innerhalb des als Handgriff ausgebildeten Transformatorgehäuses hinderlich bei der Handhabung der erfindungsgemäßen Vorrichtung ist.

Die Steuereinrichtung ist an eine elektrische Energiequelle anschließbar, damit die erfindungsgemäße Vorrichtung mit der für den Betrieb notwendigen elektrischen Energie versorgt werden kann. Dabei ist insbesondere bei einer innerhalb des als Handgriff ausgebildeten Transformatorgehäuses angeordneten Steuereinrichtung eine Energiequelle in Form von Batterien oder Akkumulatoren, die ebenfalls im Transformatorgehäuse untergebracht, aber auch außerhalb des Transformatorgehäuses angeordnet sein können. Dies ist insbesondere deshalb sinnvoll, da die gesamte erfindungsgemäße Vorrichtung dann unabhängig von einer stationären Energiequelle und insbesondere unabhängig von einem öffentlichen beziehungsweise nichtöffentlichen elektrischen Netz betrieben werden kann. Allerdings ist es natürlich auch denkbar, als Energiequelle eine stationäre Energiequelle oder ein öffentliches beziehungsweise nichtöffentliches elektrischen Netz vorzusehen, mit welcher die Steuereinheit verbindbar ist.

Um abermals die Gefahr von Funkenüberschlägen zwischen Primär und Sekundärspule zu minimieren, weisen die Primärspule und die Sekundärspule die gleiche Länge auf. Somit liegen Sekundär- und Primärspule über ihre gesamte Länge direkt gegenüber, wobei sich natürlich gemäß der Erfindung bei größerer Potentialdifferenz beziehungsweise Spannung zwischen Primär- und Sekundärspule sich deren Abstand vergrößert.

Besonders vorteilhaft hat sich dabei erwiesen, dass die Primärspule konisch koaxial um die Sekundärspule angeordnet ist. Durch eine konisch koaxiale Anordnung der Primärspule um die Sekundärspule vergrößert sich zudem der Abstand über die Länge der Spulen kontinuierlich linear, was auch dem Spannungsanstieg innerhalb der Spulen entspricht.

Durch die koaxiale Anordnung der Primärspule um die Sekundärspule erstreckt sich die Primärspule über den gesamten Bereich der Sekundärspule und es ergibt sich somit eine Abschirmung der Sekundärspule zur Umwelt. Dadurch kommt es nicht zu einer unerwünschten Verstimmung des Schwingkreises durch äußere Umwelteinflüsse, gegebenenfalls auch durch den Anwender selbst, wie dies im Stand der Technik der Fall ist.

Damit sich zwischen Primär- und Sekundärspule eine besonders gute magnetische Kopplung und somit eine besonders effektive Erzeugung der hochfrequenten Hochspannung durch den Transformator ergibt, hat es sich-bewährt, die Sekundärspule um einen Stabkern, der vorzugsweise aus einem Ferrit besteht, anzuordnen. Insbesondere die Ausbildung des Stabkerns aus einem Ferrit zeigt sich hierbei als besonders vorteilhaft, da hierdurch eine besonders gute magnetische Kopplung zwischen Primär- und Sekundärspule erreicht werden kann.

Nach einer besonders vorteilhaften Ausgestaltung der Erfindung weist die Sekundärspule eine Mehrzahl von Kammern auf, die vorzugsweise äquidistant beabstandet sind und jeweils zwischen 100 und 1000, vorzugsweise zwischen 250 und 750, besonders bevorzugt 500 Windungen aufweisen. Durch diese Maßnahme kann zum einen in einfacher Weise erreicht werden, dass der Spannungsanstieg über die Länge der Spulen besonders gleichmäßig verläuft und somit ein homogener Verlauf der hochfrequenten Hochspannung erzielt werden kann. Zum anderen lässt sich eine Sekundärspule durch eine Vielzahl von in Serie geschalteten Einzelspulen darstellen, so dass in derselben erfindungsgemäßen Vorrichtung verschiedenste Primär- und Sekundärspulenkombinationen verwirklichen werden können. Gegebenenfalls kann auch die Primärspule derart seriell aufgebaut sein, was die Kombinations- und Variationsvielfalt nochmals erhöht.

Die Sonde mit der die eigentliche Behandlung durchgeführt wird, da mit ihr das erforderliche Niederfrequenzplasma zur Anwendung auf dem zu behandelnden Gewebe erzeugt wird, ist vorzugsweise als Glassonde ausgebildet ist. Solche Glassonden sind einfach zu handhaben und in der Anwendung an oder in biologischem Gewebe physiologisch unbedenklich.

Dabei hat es sich bewährt, die Glassonde unter Unterdruck, vorzugsweise unter Unterdruck von 500 Pa bis maximal 3000 Pa, mit einem leitenden Gas, vorzugsweise mit einem Edelgas oder Edelgasgemisch zu füllen. Mit solchen leitenden Gasen, insbesondere Edelgasen und Edelgasgemischen, vorzugsweise aus Argon und/oder Neon, ist die Herstellung von Niederfrequenzplasmen und somit die gesamte erfindungsgemäße Vorrichtung besonders effizient. Die Glassonde ist an ihrem einen Ende durch einen Metallkontakt geschlossen, durch welchen die durch den Transformator gelieferte hochfrequente Hochspannung in das Innere der Glassonde geführt wird. Innerhalb der Glassonde wird das Gas dem hochfrequenten elektromagnetischen Feld ausgesetzt und somit eine Glimmentladung erzeugt. Die Leistung des Transformators ist dabei durch die Steuereinrichtung derart regelbar, dass sich Spannungen im Bereich zwischen 1800 V und 35000 V einstellen lassen, sie über das leitfähige Gas innerhalb der Glassonde auf die Behandlungsfläche der Glassonde übertragen werden. Befindet sich die Behandlungsfläche der Glassonde unmittelbar über dem zu behandelnden biologischen Gewebe stellt sich diese Spannung dazwischen ein, gegebenenfalls in Abhängigkeit des elektrischen Widerstandes der Oberfläche des zu behandelnden biologischen Gewebes und dem Widerstand der Gase, insbesondere der Luft, zwischen Behandlungsfläche der Glassonde und Oberfläche des zu behandelnden biologischen Gewebes.

Damit die durch den Transformator zur Verfügung gestellte hochfrequente Hochspannung auch effizient durch die Sonde genutzt werden kann, ist eine gute und sichere elektrische Kontaktierung zwischen Transformator und Sonde unabdingbar. Dies wird nach einen eigenständigen Gedanken der Erfindung dadurch erreicht, dass die Sonde mittels einer Kontaktfeder elektrisch/elektronisch mit dem Transformator koppelbar ist. Dabei ist es zum einen denkbar, dass die Kontaktfeder an dem Transformator beziehungsweise dem Transformatorgehäuse angeordnet ist. Zum anderen kann die Kontaktfeder auch an der Sonde angeordnet sein. In beiden Fällen stellt die Kontaktfeder die elektrische Kontaktierung zwischen Sonde und Transformator sicher, auch wenn innerhalb der Kupplung zwischen Sonde und Transformator ein nicht erwünschtes Spiel auftritt.

Ein beispielhaftes Verfahren zur Behandlung von biologischem Gewebe mit einem Niederdruckplasma mit einer zuvor beschrieben Vorrichtung enthält im Wesentlichen folgende Verfahrensschritten:

a) Bereitstellen elektrischer Energie in Form von elektrischer Gleichspannung oder niederfrequenter Wechselspannung im Bereich von 12 V bis 600 V mit einer Stromstärke auf der Seite der Sekundärspule von 0,1 $\mu$A bis 300 $\mu$A,
b) Umwandlung der elektrischen Gleichspannung oder der elektrischen niederfrequenten Wechselspannung in hochfrequente Wechselspannung zwischen 10 kHz und 50 kHz,
c) Transformation der hochfrequenten Wechselspannung in einen Spannungsbereich zwischen 1800 V bis 35000 V,
d) Weiterleitung der hochfrequenten Wechselspannung in einen Spannungsbereich zwischen 1800 V bis 35000 V an eine Sonde (2), vorzugsweise eine Glassonde, welche über dem zu behandelnden biologischen Gewebe in einem Abstand zwischen 1 mm und 5 cm positioniert ist.

Hierbei sei darauf hingewiesen, dass bei Anwendungen im Dentalbereich, z. B. bei der Behandlungen der Mundschleimhäute in der Mundhöhle, die Stromstärke auf der Seite der Sekundärspule zwischen 0,1 $\mu$A und 100 $\mu$A gewählt wird, während bei Anwendungen auf sonstigen Gewebeoberflächen, insbesondere dermatologischen Behandlungen der übrigen Haut beziehungsweise des zu behandelten Patienten beziehungsweise gynäkologischen Anwendungen, die Stromstärke auf der Seite der Sekundärspule zwischen 0,1 $\mu$A und 300 $\mu$A gewählt wird.

Weitere Ziele, Vorteile, Merkmale und Anwendungsmöglichkeiten der vorliegenden Erfindung ergeben sich aus der nachfolgenden Beschreibung von Ausführungsbeispielen anhand der Zeichnungen.

Es zeigen:

Figur 1a:       eine aus dem Stand der Technik bekannte Vorrichtung zur Behandlung von biologischem Gewebe mit Ozon in der Hand eines Benutzers,
Figur 1 b:       eine Ersatzschaltbild der Vorrichtung gemäß Figur 1 b,
Figur 2:       ein Transformators eines Ausführungsbeispiel einer erfindungsgemäßen Vorrichtung in einem Transformatorgehäuse,
Figur 3:       ein Transformatorgehäuse eines Ausführungsbeispiel einer erfindungsgemäßen Vorrichtung,
Figuren 4a-i:       verschiedene Ausführungsformen einer Sonde eines Ausführungsbeispiel einer erfindungsgemäßen Vorrichtung,

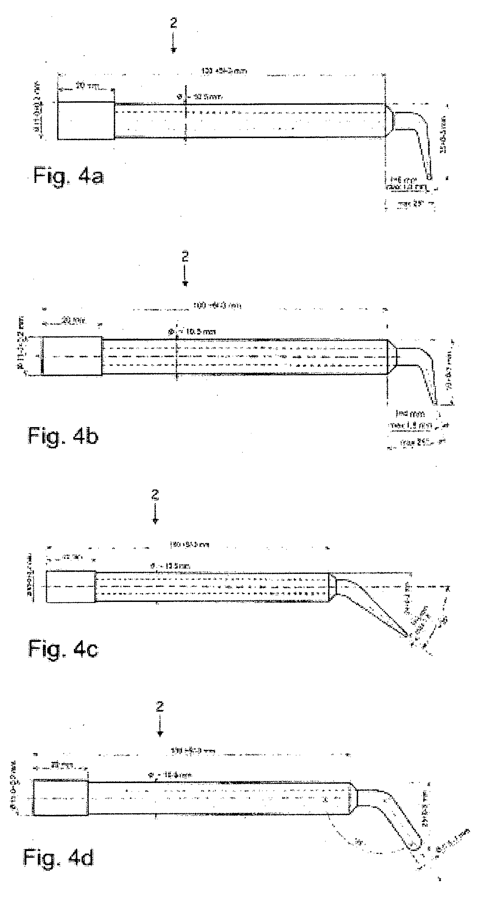

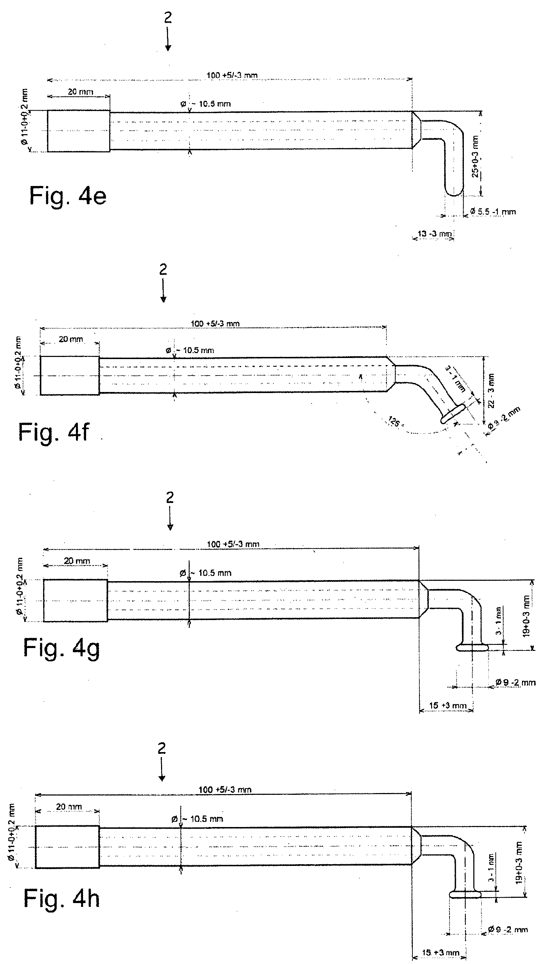

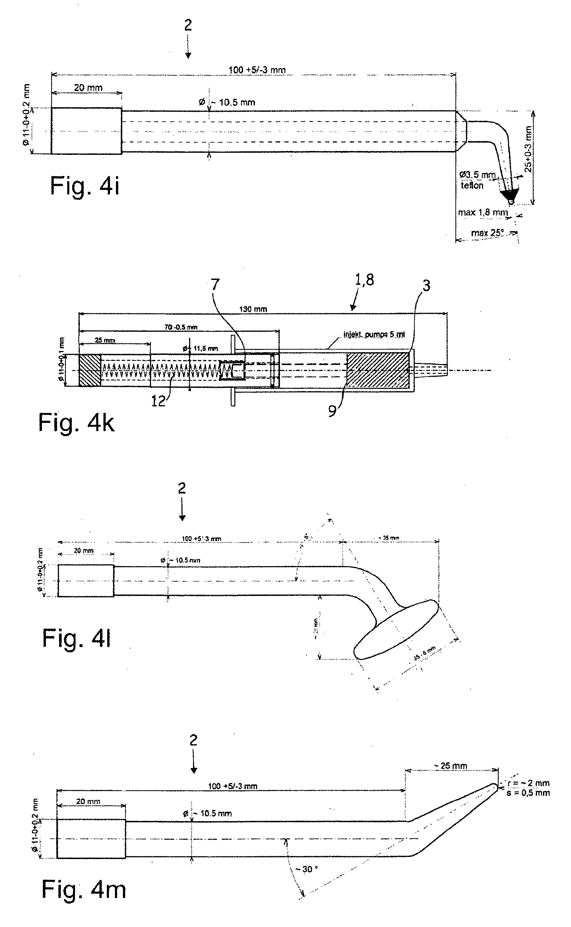

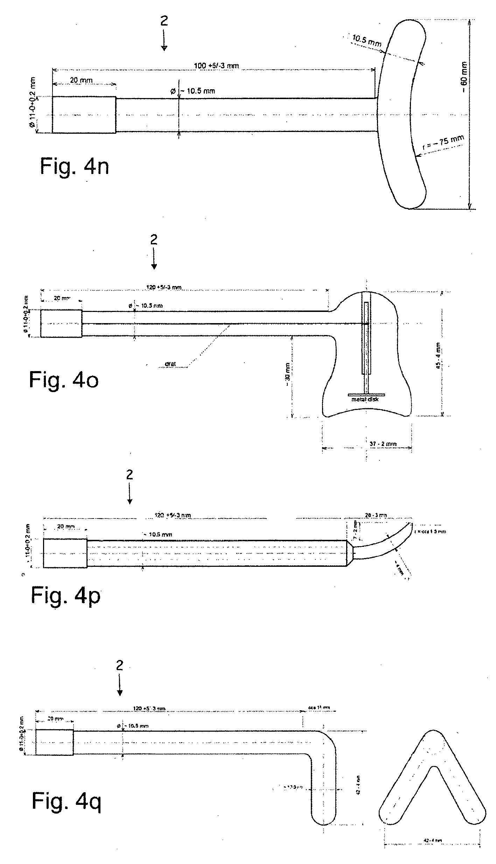

Figur 4 k: eine Transformatorgehäuse mit Transformator und Steuereinrichtung eines Ausführungsbeispiel einer erfindungsgemäßen Vorrichtung zum Anschluß einer Sonde der Figuren 4a bis i und 4l bis q,

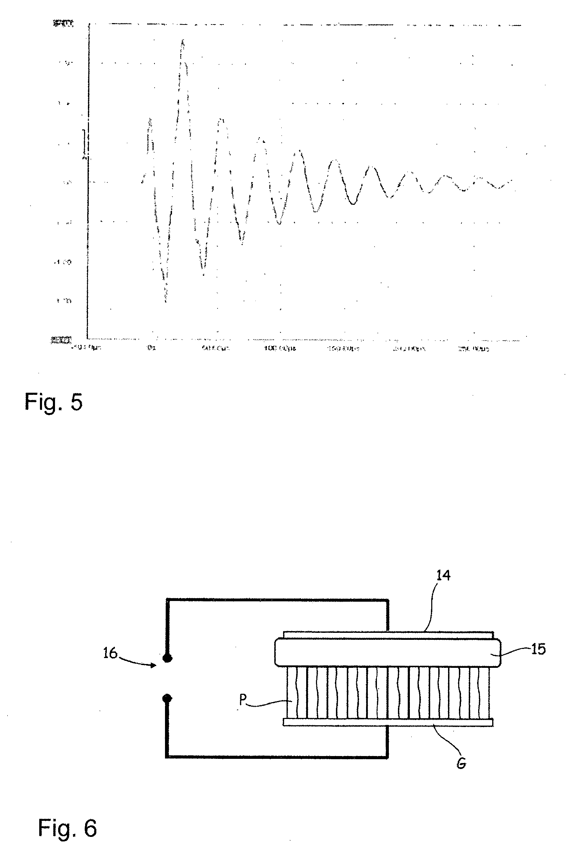

Figur 5: typischer Pulsverlauf eines hochfrequenten Spannungspulses, bei dem die Stromstärke in μA gegen die Zeit aufgetragen ist und

Figur 6: schematische Darstellung einer dielektrischen Barriereentladung.

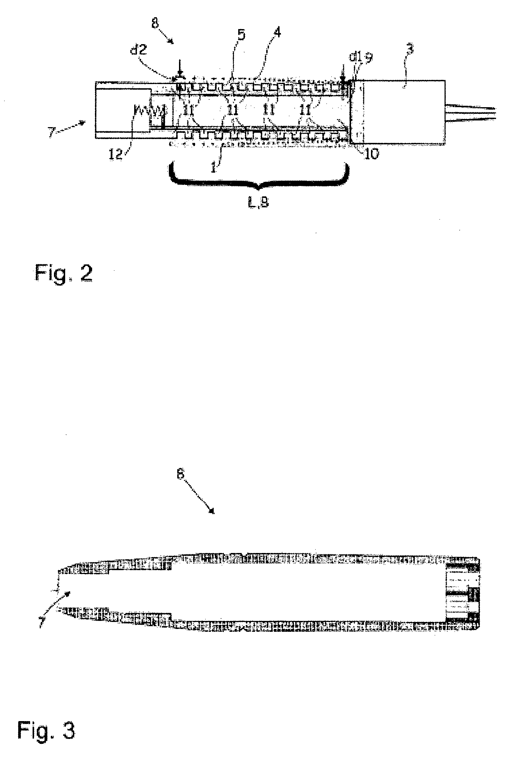

In den Figuren 2, 3 und 4a bis q sind verschiedene Elemente von Ausführungsformen erfindungsgemäßer Vorrichtungen zur Behandlung von biologischem Gewebe mit einem Niederdruckplasma dargestellt, welche weiter unten näher erläutert werden.

Figur 2 zeigt beispielsweise eine Ausführungsform eines Transformatorgehäuses 8 einer erfindungsgemäßen Vorrichtung, in welches ein aus einer Primärspule 4 und einer Sekundärspule 5 gebildeter Transformator angeordnet ist, an den wiederum über eine Kupplung 9 eine Steuereinrichtung 3 angeschlossen ist. Die Steuereinrichtung 3 ist wiederum mit einer hier nicht dargestellten elektrischen Energiequelle 13 zur Einspeisung elektrischer Energie in den Transformator 1 verbunden. An dem der Kupplung 9 gegenüberliegenden Ende des Transformatorgehäuses 8 ist wiederum eine Kupplung 7 angeordnet, an welcher eine Sonde 2, vorzugsweise eine Glassonde angeordnet werden kann. Eine Kontaktfeder 12 stellt hierbei sicher, dass zwischen dem Transformator 1 und der Sonde 2 immer ein elektrischer Kontakt besteht. Das Transformatorgehäuse 8 ist vorliegend als ein Handgriff ausgebildet und erstreckt sich in seiner Längserstreckung in die gleiche Richtung wie die Primärspule 4 und die Sekundärspule 5.

Die Sekundärspule 5 ist in diesem Ausführungsbeispiel um einen Stabkern 10, der vorzugsweise aus einem Ferrit besteht, gewickelt, während die Primärspule 4 in einem Abstand um die Sekundärspule 5 gewickelt ist. Dieser Abstand nimmt dabei von dem der Kupplung 9 zugewandten Ende der Spule 4 und 5 von einem Abstand d1 bis zu dem der Kupplung 7 zugewandten Ende der Spulen 4 und 5 kontinuierlich bis zu einem Abstand d2 zu, so dass die Primärspule 4 konisch koaxial über der Sekundärspule angeordnet ist. In vorliegenden Ausführungsbeispiel weisen beide Spulen 4 und 5 die gleiche Länge L auf, so dass sie über ihre gesamte Länge einen Überlappungsbereich B bilden. Die Primärspule 4 nimmt dabei auch die Funktion eines elektromagnetischen Schirms wahr, beziehungsweise gewährleistet einen Abschirmeffekt, durch den elektromagnetische Störfelder nicht das durch den Transformator 1 erzeugte hochfrequente elektromagnetische Feld entscheidend stören können, so dass eine einwandfreie Funktion der erfindungsgemäßen Vorrichtung gegeben ist. Daneben können in einem Endabschnitt des Konverters noch Dichtungseinrichtungen vorgesehen sein.

Der als Hochspannungstransformator ausgebildete Transformator 1 ist in diesem Ausführungsbeispiel derart aufgebaut, dass die innere Sekundärspule 5 um einen Stabkern 10 aus Ferrit in Kammern 11 gewickelt ist. Bei der hier gezeigten Ausführungsform weist Sekundärspule 5 je 500 Windungen pro Kammer 11 auf; es sind jedoch auch andere Windungszahlen denkbar.

Der Transformator 1 nimmt einerseits die Aufgabe wahr, die von der Energiequelle 13 und der Steuereinheit 3 bereitgestellte hochfrequente Niederspannung in hochfrequente Hochspannung umzuwandeln. Andererseits nimmt er aber auch die Aufgabe wahr, die erzeugte Hochspannung, insbesondere über eine hier nicht dargestellte innere Glasröhre der als Glassonde ausgebildeten Sonde 2 an deren Behandlungsfläche zu leiten, die an dem Kupplung 7 gegenüberliegenden Ende der Sonde angeordnet ist..

Die Anordnung der Spulen 4 und 5 innerhalb des Transformators 1 führt zur Bereitstellung von Pulsen mit einer vorgegebenen Signalform, bevorzugt von sinusförmigen Pulsen und besonders bevorzugt von exponentiell gedämpften sinusförmigen Pulsen, wie sie beispielhaft in Figur 5 dargestellt sind und mit welchen ein kaltes Plasmas beziehungsweise ein Niederdruckplasma zwischen Behandlungsfläche der Sonde 2 und zu behandelnden Gewebe erzeugt werden kann.

Figur 3 zeigt den Aufbau eines Transformatorgehäuses 8 der Figur 2, welches aus einem elektrisch isolierendem Material, vorzugsweise einem Kunststoff hergestellt ist..

Die Figuren 4a-i und 4l-q zeigen 15 verschiedene Beispiele als Glassonden ausgebildeter Sonden 2, deren Behandlungsfläche je nach zu behandelndem biologischem Gewebe G schräg oder plan oder gebogen ausgeführt ist.

An dem die Kupplung 7 für die Sonde 2aufweisneden Ende des Transformatorgehäuses 8 ist dieses mit einer Kontaktfeder 12 ausgestattet, welche elektronisch mit dem Transformator 1 verbunden ist. Wie bereits kurz erwähnt, stellt die Kontaktfeder 12 den Kontakt mit der Sonde 2 her. Durch den Kontakt werden die Spannungsimpulse auf die Sonde 2 übertragen. Die als Glassonde ausgebildete Sonde 2 ist in den Ausführungsbeispielen der Figuren 4a-i und 4l-q mit zwei Kammern ausgestattet. Die innere Kammer ist bevorzugt mit 100 % Neon gasgefüllt bei einem Unterdruck von 500 Pa bis 3000 Pa und leitet die Hochspannung an die Spitze der Instrumentensonde. Die äußere Kammer dient zur Isolierung und Schutz der inneren Kammer. Die innere Kammer besteht vorteilhaft aus Glas und die äußere Kammer kann aus dem Material Glas oder Edelmetall bestehen.

An dem der Behandlungsfläche gegenüberliegenden Ende ist die Sonde 2 mit einer Metallklappe geschlossen, welche zusammen mit der Kontaktfeder 12 und der Kupplung 7 die elektrische Steckverbindung mit dem in dem Transformatorgehäuse 8 angeordneten Transformator 1 herstellt.

Zwischen dem Behandlungsfläche der Sonde 2 und dem zu behandelnden biologischen Gewebe G bildet sich bei einem Abstand zwischen 1 mm und 5 mm durch die bereitgestellte hochfrequente Wechselspannung und dem typischen Pulsverlauf die Bildung des kalten Plasmas beziehungsweise des Niederdruckplasmas, mit welchem Bakterien, Keimen, Viren, Pilzen oder andere vergleichbare, dem Gewebe G anhaftenden Mikroorganismen abgetötet werden können.

Das Gas in der als Glassonde ausgebildeten Sonde 2 wird dabei dem erzeugten hochfrequenten, elektromagnatischen Wechselfeld ausgesetzt, um eine Glimmentladung (Mikroentladung) zu erzeugen. Die Leistung des Transformators 1 ist dabei über die Steuereinrichtung 3 so regelbar, dass sich Spannungen im Bereich zwischen 1,8 und 35 kV einstellen lassen, die über das leitfähige Gas auf die Behandlungsfläche der Sonde 2 übertragen werden. Befindet sich die Behandlungsfläche der Sonde 2 unmittelbar über dem zu behandelnden Gewebe G, stellt sich deren Spannung in Abhängigkeit von Hautwiderstand der Luft zwischen Instrumentensondenspitze und Hautoberfläche ein.

Das Verfahren zur direkten Erzeugung eines Niederdruckplasmas oder kalten Plasmas entspricht dem Aufbau der in Figur 6 dargestellten dielektrischen Barreeentladung. Die Anregungsspannung wird im Transformator 1 erzeugt. Die Sonde 2 bildet dabei eine Metallelektrode 14 und ein und Dielektrikum 15. Die Erdelektrode wird von dem zu behandelnden Gewebe G gebildet, so dass zwischen dem Gewebe G und der Metallelektrode 14 der Sonde 2 im Wesentlichen die durch den Transformator 1 gelieferte hochfrequente Anregungsspannung 16 anliegt. Das dargestellte Schema dient als Modell für die weiteren Betrachtungen.

Physikalische Betrachtung der Plasmabildung durch dielektrische Barriereentladung. Die dielektrische Barriereentladung, auch dielektrisch behinderte Entladung oder stille Entladung genannt, ruft bei Atmosphärendruck während der Zündphase nicht-thermische Plasmafilamente P hervor. Die dielektrisch behinderte Entladung oder stille Entladung ist in dieser Betrachtung neben der Koronaentladung eine Variante der Gasentladungen, die bei Atmosphärendruck während der Zündphase nicht-thermische Plasmafilamente P hervorruft. Der Unterschied zwischen beiden Gasentladungsformen liegt im Löschmechanismus der Entladungsfilamente. Im Fall der Koronaentladung ist er raumladungsorientiert und bei der Barriereentladung oberflächenladungsorientiert.

Der in Figur 6 dargestellte, grundsätzliche Aufbau besteht aus zwei Elektroden, einer Hochspannungselektrode 14 und einer Erdelektrode G, mit ein oder mehreren dielektrischen Barrieren 15 (Isolatoren) dazwischen. Zwischen Dielektrikum 15 und Erdelektrode G befindet sich ein Spalt, der in der Breite variabel ist, in der Größenordnung von einigen mm bis in den cm-Bereich. Die zu behandelnde Probe befindet sich auf der beziehungsweise bildet die Erdelektrode G. Um die Entladung zu erzeugen, wird eine Wechselspannung von 1-100 kV und Frequenzen von 10-50 kHz benötigt. Diese Entladung ist charakterisiert durch die Ausbildung von Mikroentladungen beziehungsweise Plasmafilamente P. Bei dieser Reaktion lagern sich Ladungsträger an die Oberfläche des Dielektrikums 15 an und schwächen das externe elektrische Feld, was zu einem Auslöschen der Plasmafilamente P führt. Das Dielektrikum 15 dient der Strombegrenzung und es ermöglicht, dass die Entladungen an einer Vielzahl statistisch gleichverteilter Punkte stattfinden können, womit eine flächige Plasmabehandlung der gesamten Oberfläche des zu behandelnden Gewebes G ermöglicht wird.

Die physikalische Betrachtung der Plasmabildung erfolgt nach der Methode von Paschen und Townsend. Die Analyse bezieht sich auf das in Figur 6 dargestellte Modell für die dielektrische Barriereentladung. Die Betrachtung ermöglicht die Ermittlung der Durchbruchspannung (= Zündspannung), die zur Bildung eines Plasmas führt. Unterhalb der Durchbruchspannung liegen Plasmafilamente P vor, die charakteristisch für ein kaltes Plasma beziehungsweise Niederdruckplasma sind.

Ausgangspunkt ist ein Kondensator mit einem Plattenabstand von d=1mm . Zwischen seinen Platten befinden sich Luft. Es sei $\alpha$ die Wahrscheinlichkeit pro Längeneinheit, dass ein Elektron ein neutrales Atom bzw. Molekül ionisiert, wobei Stöße von Ionen mit Neutralatomen aufgrund des schnell wechselnden Feldes und der großen Masse der Ionen vernachlässigt werden können.

Wenn N die Anzahl der entstandenen Elektronen ist, dann gilt:

$$dN/dx = \alpha N \quad (1.1)$$

$$\Rightarrow N(d) = N_0 \mathrm{e}^{\alpha d} \quad (1.2)$$

Dabei ist $N_0$ die Anzahl extern erzeugter Elektronen, beispielsweise durch kosmische Strahlung. Die Zahl ionisierender Stöße ist proportional zum Druck p und zur Wahrscheinlichkeit für einen Ionisationsstoß.

Außerdem gilt für die kinetische Energie der Elektronen

$$E_{ion} = eE\lambda_{ion} \quad (1.3)$$

Dabei ist $\lambda_{ion}$ die Beschleunigungsstrecke und E die angelegte elektrische Feldstärke. Aufgrund inelastischer Stöße durchläuft nur ein Bruchteil

$$\exp\left(\frac{\lambda_{ion}}{\lambda_{inel}}\right)$$

die Strecke $\lambda_{ion}$ ohne Energieverlust.

Es folgt für die Konstante

$$\alpha = Ap\, e^{-\left(\frac{\lambda_{ion}}{\lambda_{inel}}\right)} = Ap\exp\left(-\left(B\frac{p}{E}\right)\right) \quad (1.4)$$

Mit der Durchbruchspannung $U_{zünd} = E_d$ ergibt sich

$$U_{zünd} = \frac{Bpd}{\ln(Apd) - \ln(\ln(1+\gamma^{-1}))} \approx 3kV \quad (1.5)$$

Dabei ist $\gamma$ die Zahl der erzeugten Elektronen pro Ion (dritter Townsend-Koeffizient), mit der die Zündbedingung

$$\gamma\left(e^{\alpha d} - 1\right) \geq 1 \quad (1.6)$$

lautet. Dabei gilt in der Regel $\gamma \ll 1$

Paschenkurve für Luft (Kurve 1) und SF6 (Kurve 2).
p: Druck,
s: Spaltgröße.

Die Paschenkurve beschreibt die Abhängigkeit der Durchbruchspannung für die Erzeugung einer Gasentladung vom Produkt aus Spaltgröße und Druck.

Für den vorliegenden Fall kann die Abhängigkeit der Durchbruchspannung von der Spaltbreiten abgeschätzt werden.

| Spaltbreite | $U_{zünd}$ |
|---|---|
| 1 mm | 3 kV |

(fortgesetzt)

| Spaltbreite | $U_{zünd}$ |
|---|---|
| 2 mm | 6 kV |
| 3 mm | 9 kV |
| 4 mm | 12 kV |
| 5 mm | 15 kV |
| 6 mm | 18 kV |

Bei einer Spannung von 3 kV setzt also für Luft bei 1 bar der elektrische Durchbruch ein. Da hier auf der gesamten Strecke d alle Atome bzw. Moleküle ionisiert sind, ist dies die Obergrenze für diejenige Spannung, die für ein stabiles Plasma nötig ist. Unterhalb dieser Spannung bilden sich in einer Barriereentladung dünne Entladungskanäle (Plasmafilamente P) zwischen den Elektroden (Abstand im Bereich 1 mm) aus, die charakteristisch für ein kaltes Plasma sind. Bei Atmosphärendruck wird statistisch verteilt eine hohe Zahl von kurzlebigen Entladungskanälen (Mikroentladungen) beobachtet.

Ein notwendiges Kriterium für die Existenz eines Plasmas ist, dass die Debye-Länge klein gegenüber den Abmessungen des Systems ist. Diese Abschirmlänge ist dadurch charakterisiert, dass auf dieser das Potential einer lokalen Ionen- oder Elektronenladung hinreichend stark (in der Regel auf das 1/e-fache) abgefallen ist. Dies rührt daher, dass in einem Plasma ein positives Ion von einer kugelförmigen Wolke aus Elektronen umgeben ist, so dass sich die Ladungen in etwa kompensieren, wobei der Radius dieser Kugel die Debye-Länge ist. Im vorliegenden Fall ist die Bewegung der Ionen im Wechselfeld gegenüber derjenigen der Elektronen aufgrund der viel größeren Ionenmasse zu vernachlässigen. Deshalb gilt für die Debye-Länge:

$$\lambda_d = \sqrt{\frac{\epsilon_0 k_b T_e}{n_e e^2}} \quad (2.1)$$

Für ein nicht-isothermes Plasma, wobei die Elektronen aufgrund ihrer kleineren Masse eine höhere Temperatur haben als die Ionen, bei einer Barriereentladung

$$T_e \sim 1 - 10\,eV \qquad (2.2)$$

(Elektronentemperatur) und

$$n_e \sim 10^{20} - 10^{21}\,m^{-3} \qquad (2.3)$$

(Volumenanzahldichte der Elektronen).

Setzt man diese Werte in die Gleichung (2.1) ein, so erhält man für die Debye-Länge eines nichtisothermen Plasmas einer Barriereentladung

$$\lambda_d = 2.35 \cdot 10^{-6}\,m \quad (2.4),$$

wobei diese Debye-Länge für den ungünstigsten Fall einer Anzahldichte von $n_e$=1020 m$^{-3}$ und einer Elektronentemperatur von Te=10 eV=1,16·105 K berechnet wurde.

Geht man für den vorliegenden Fall davon aus, dass das System von einer Größenordnung im mm-Bereich ist, dann ist die Debye-Länge um einen Faktor 1000 kleiner, womit das notwendige Kriterium für die Existenz eines Plasmas erfüllt ist.

Ein weiteres Kriterium ist, dass die mittlere Anzahl geladener Teilchen in der Debye-Kugel größer als eins ist. Im ungünstigsten Fall $n_e$=1020 m$^{-3}$ befinden sich in der Debye-Kugel etwa 5000 geladene Teilchen, womit auch dieses Kriterium erfüllt ist.

Die Parameter der erfindungsgemäßen Vorrichtung erfüllen die physikalischen Voraussetzungen zur Erzeugung eines kalten Plasmas.

| physikalischer Parameter | notwendige Bedingung | plasmaOne | notwendige Bedingung erfüllt? |
|---|---|---|---|
| Durchbruchspannung | 3 kV bei 1 mm Spalt | 3 bis 18 kV | ja |
| Debye-Länge | Spaltgröße >> $\lambda_d$=2.35·10$^{-6}$ $m$ | Spaltgroße >= 1 mm | ja |
| mittlere Anzahl geladener Teilchen in Debye-Kugel | Anzahl > 1 | Anzahl: ca 5000 | ja |

**Bezugszeichenliste**

1    Transformator
2    Sonde
3    Steuereinrichtung
4    Primärspule
5    Sekundärspule
7    Kupplung
8    Transformatorgehäuse
9    Kupplung
10   Stabkern
11   Kammer
12   Kontaktfeder
13   Energiequelle
14   Metallelektrode
15   Dielektrikum
16   Anregungsspannung
P    Plasmafilemente
B    Überlappungsbereich
d1   Abstand
d2   Abstand
F    Finger
K    Gesamtkapazität
CF   Kapazität Finger
L    Länge
SK   Schwingkreis
G    Gewebe

**Patentansprüche**

1. Vorrichtung zur Behandlung von biologischem Gewebe (G) mit einem Niederdruckplasma mit

a) einem Transformator (1) zur Erzeugung eines hochfrequenten elektromagnetischen Feldes,
b) einer Sonde (2), welche mit dem Transformator (1) elektrisch koppelbar ist und
c) einer Steuerungseinrichtung (3) zur Steuerung des durch den Transformator (1) erzeugten hochfrequenten elektromagnetischen Feldes,

wobei der Transformator (1) eine Primärspule (4) und eine koaxial dazu angeordnete Sekundärspule (5) aufweist, **dadurch gekennzeichnet, dass** sich der Zwischenraum zwischen Primärspule (4) und Sekundärspule (5) im Überlappungsbereich (B) der beiden Spulen (4, 5) von einem ersten Abstand (d1) zu einem zweiten größeren Abstand (d2) in Richtung einer Kupplung (7) für die Sonde (2) vergrößert, wobei die Primärspule konisch koaxial über der Sekundärspule angeordnet ist.

**2.** Vorrichtung nach Anspruch 1,
**dadurch gekennzeichnet, dass**
der Transformator (2) ein Transformatorgehäuse (8) umfasst, welches eine der Kupplung (7) gegenüberliegende Kupplung (9) zum elektrischen/elektronischem Anschluss der Steuereinrichtung (3) aufweist, wobei das Transformatorgehäuse (8) vorzugsweise als Handgriff ausgebildet und entsprechend ergonomisch geformt ist.

**3.** Vorrichtung nach Anspruch 2,
**dadurch gekennzeichnet, dass**
die Steuereinrichtung (3) in dem Transformatorgehäuse (8) angeordnet ist.

**4.** Vorrichtung nach Anspruch 1 oder 2,
**dadurch gekennzeichnet, dass**
die Steuereinrichtung (3) außerhalb des Transformatorgehäuse (8) angeordnet ist.

**5.** Vorrichtung nach einem der vorhergehenden Ansprüche,
**dadurch gekennzeichnet, dass**
an die Steuereinrichtung (3) eine elektrische Energiequelle anschließbar ist.

**6.** Vorrichtung nach einem der vorhergehenden Ansprüche,
**dadurch gekennzeichnet, dass**
die Primärspule (4) und die Sekundärspule (5) die gleiche Länge (L) aufweisen.

**7.** Vorrichtung nach einem der vorhergehenden Ansprüche,
**dadurch gekennzeichnet, dass**
die Sekundärspule (5) um einen Stabkern (10), der vorzugsweise aus einem Ferrit besteht, angeordnet ist.

**8.** Vorrichtung nach einem der vorhergehenden Ansprüche,
**dadurch gekennzeichnet, dass**
die Sekundärspule eine Mehrzahl von Kammern (11) aufweist, die vorzugsweise äquidistant beabstandet sind und jeweils zwischen 100 und 1000, vorzugsweise zwischen 250 und 750, besonders bevorzugt 500 Windungen aufweisen.

**9.** Vorrichtung nach einem der vorhergehenden Ansprüche,
**dadurch gekennzeichnet, dass**
die Sonde (2) als Glassonde ausgebildet ist.

**10.** Vorrichtung nach Anspruch 9,
**dadurch gekennzeichnet, dass**
die Glassonde unter Unterdruck, vorzugsweise unter Unterdruck von 500 Pa bis 3000 Pa, besonders bevorzugt von 2000 Pa, mit einem leitenden Gas, vorzugsweise mit einem Edelgas oder Edelgasgemisch gefüllt ist.

**11.** Vorrichtung nach einem der vorhergehenden Ansprüche,
**dadurch gekennzeichnet, dass**
die Sonde (2) mittels einer Kontaktfeder (12) elektrisch/elektronisch mit dem Transformator (1) koppelbar ist, wobei die Kontaktfeder (12) entweder an dem Transformator (1) oder an der Sonde (2) angeordnet ist.

**Claims**

**1.** Apparatus for treating biological tissue (G) using a low-pressure plasma, comprising

a) a transformer (1) for generating a high-frequency electromagnetic field,
b) a probe (2) which is able to be electrically coupled to the transformer (1), and
c) a control device (3) for controlling the high-frequency electromagnetic field generated by the transformer (1),

wherein the transformer (1) has a primary coil (4) and a secondary coil (5) arranged coaxially thereto, **characterized in that** the intermediate space between the primary coil (4) and the secondary coil (5) in the overlap area (B) of the two coils (4, 5) increases from a first distance (d1) to a second, greater distance (d2) in the direction of a coupling (7) for the probe (2), wherein the primary coil is arranged in a conical and coaxial manner over the secondary coil.

2. Apparatus according to claim 1, **characterized in that** the transformer (2) comprises a transformer housing (8) which has a coupling (9), opposite the coupling (7), for the electrical/electronic connection of the control device (3), wherein the transformer housing (8) is preferably configured as a candle and is accordingly shaped in an ergonomic manner.

3. Apparatus according to claim 2, **characterized in that** the control device (3) is arranged in the transformer housing (8).

4. Apparatus according to claim 1 or 2, **characterized in that** the control device (3) is arranged outside the transformer housing (8).

5. Apparatus according to any of the preceding claims, **characterized in that** an electrical energy source is able to be connected to the control device (3).

6. Apparatus according to any of the preceding claims, **characterized in that** the primary coil (4) and the secondary coil (5) have the same length (L).

7. Apparatus according to any of the preceding claims, **characterized in that** the secondary coil (5) is arranged around a rod core (10) which is preferably made of a ferrite.

8. Apparatus according to any of the preceding claims, **characterized in that** the secondary coil has a plurality of chambers (11) which are preferably equidistantly spaced apart and each have between 100 and 1000, preferably between 250 and 750, particularly preferably 500 turns.

9. Apparatus according to any of the preceding claims, **characterized in that** the probe (2) is configured as a glass probe.

10. Apparatus according to claim 9, **characterized in that** the glass probe is filled with a conductive gas, preferably with a noble gas or a noble gas mixture, under negative pressure, preferably under a negative pressure of 500 Pa to 3000 Pa, particularly preferably 2000 Pa.

11. Apparatus according to any of the preceding claims, **characterized in that** the probe (2) is able to be electrically/electronically coupled to the transformer (1) by means of a contact spring (12), wherein the contact spring (12) is arranged either on the transformed (1) or on the probe (2).

**Revendications**

1. Dispositif de traitement d'un tissu biologique (G) au moyen d'un plasma basse pression comprenant

a) un transformateur (1) servant à générer un champ électromagnétique haute fréquence,
b) une sonde (2), laquelle peut être couplée électriquement au transformateur (1) et
c) un dispositif de commande (3) servant à commander le champ électromagnétique haute fréquence généré par le transformateur (1),

le transformateur (1) comprenant une bobine primaire (4) et une bobine secondaire (5) agencée coaxialement à cette dernière,
**caractérisé en ce que** l'espace intermédiaire entre la bobine primaire (4) et la bobine secondaire (5) s'agrandit dans une zone de chevauchement (B) des deux bobines (4, 5) d'un premier écartement (d1) à un deuxième écartement (d2) en direction d'un couplage (7) destiné à la sonde (2), la bobine primaire étant agencée de manière conique et coaxiale au-dessus de la bobine secondaire.

2. Dispositif selon la revendication 1,

**caractérisé en ce que**
le transformateur (2) comprend un boîtier (8) du transformateur, lequel comprend un couplage (9) opposé au couplage (7) pour le raccordement électrique/électronique du dispositif de commande (3), le boîtier (8) du transformateur est réalisé de préférence sous la forme d'une poignée et est formé en conséquence de manière ergonomique.

3. Dispositif selon la revendication 2,
   **caractérisé en ce que**
   le dispositif de commande (3) est agencé dans le boîtier (8) du transformateur.

4. Dispositif selon la revendication 1 ou 2,
   **caractérisé en ce que**
   le dispositif de commande (3) est agencé à l'extérieur du boîtier (8) du transformateur.

5. Dispositif selon l'une quelconque des revendications précédentes,
   **caractérisé en ce que**
   une source d'énergie électrique peut être raccordée au dispositif de commande (3).

6. Dispositif selon l'une quelconque des revendications précédentes,
   **caractérisé en ce que**
   la bobine primaire (4) et la bobine secondaire (5) présentent la même fongueur (L).

7. Dispositif selon l'une quelconque des revendications précédentes,
   **caractérisé en ce que**
   la bobine secondaire (5) est agencée autour d'un noyau en forme de barre (10), qui est constitué de préférence d'une ferrite.

8. Dispositif selon l'une quelconque des revendications précédentes,
   **caractérisé en ce que**
   la bobine secondaire comprend une pluralité de chambres (11), qui sont de préférence espacées les unes des autres de manière équidistante et comprennent chacune entre 100 et 1000 spires, de préférence entre 250 et 750 spires, de manière particulièrement préférée 500 spires.

9. Dispositif selon l'une quelconque des revendications précédentes,
   **caractérisé en ce que**
   la sonde (2) est réalisée sous la forme d'une sonde en verre.

10. Dispositif selon la revendication 9,
    **caractérisé en ce que**
    la sonde en verre est remplie sous dépression d'un gaz conducteur, de préférence sous une dépression de 500 Pa à 3 000 Pa, d'une manière particulièrement préférée de 2 000 Pa, de préférence d'un gaz rare ou d'un mélange de gaz rares.

11. Dispositif selon l'une quelconque des revendications précédentes,
    **caractérisé en ce que**
    la sonde (2) peut être couplée électriquement/électroniquement au transformateur (1) au moyen d'un ressort de contact (12), le ressort de contact (12) est agencé soit sur le transformateur (1) soit sur la sonde (2).

Fig. 1a

Fig. 1b

Fig. 2

Fig. 3

**Fig. 4a**

**Fig. 4b**

**Fig. 4c**

**Fig. 4d**

Fig. 4e

Fig. 4f

Fig. 4g

Fig. 4h

Fig. 4i

Fig. 4k

Fig. 4l

Fig. 4m

Fig. 4n

Fig. 4o

Fig. 4p

Fig. 4q

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0837622 A1
- DE 102005000950 B3
- DE 3618412 A1
- WO 2006119997 A1